**Europäisches Patentamt**

**European Patent Office**

**Office européen des brevets**

(19)

(11) Veröffentlichungsnummer : **0 388 744 B1**

(12)

# EUROPÄISCHE PATENTSCHRIFT

(45) Veröffentlichungstag der Patentschrift :
**12.05.93 Patentblatt 93/19**

(51) Int. Cl.⁵ : **C07C 229/30, C07C 227/06**

(21) Anmeldenummer : **90104592.2**

(22) Anmeldetag : **10.03.90**

(54) **Verfahren zur Herstellung von beta-Amino-acrylsäureestern.**

(30) Priorität : **23.03.89 DE 3909596
23.03.89 DE 3909598**

(43) Veröffentlichungstag der Anmeldung :
**26.09.90 Patentblatt 90/39**

(45) Bekanntmachung des Hinweises auf die
Patenterteilung :
**12.05.93 Patentblatt 93/19**

(84) Benannte Vertragsstaaten :
**CH DE FR GB IT LI**

(56) Entgegenhaltungen :
**DE-A- 3 037 086
DE-A- 3 531 067
ANNALES DE CHIMIE, 10. Serie, Band 18,
1932, Action des bases sur l'éther formylacétique", Seiten 107-110**

(73) Patentinhaber : **BAYER AG
W-5090 Leverkusen 1 Bayerwerk (DE)**

(72) Erfinder : **Blank, Heinz Ulrich, Dr.
Am Geusfelde 35
W-5068 Odenthal (DE)**
Erfinder : **Wolters, Erich, Dr.
Stenzelbergstrasse 11
W-5000 Köln 41 (DE)**
Erfinder : **Ullrich, Friedrich-Wilhelm, Dr.
Jakob-Böhme-Strasse 11
W-5000 Köln 80 (DE)**
Erfinder : **Kraus, Helmut, Dr.
Wolfskaul 2
W-5000 Köln 80 (DE)**
Erfinder : **Marzolph, Gerhard, Dr.
Semmelweisstrasse 87a
W-5000 Köln 80 (DE)**
Erfinder : **Silber, Gunter, Dr.
Gerstenkamp 21
W-5000 Köln 80 (DE)**

## Beschreibung

Die vorliegende Erfindung betrifft ein Verfahren zur Herstellung von β-Amino-acrylsäureestern aus β-Hydroxy-acrylsäureester-alkalisalzen und Ammoniumsalzen in einem aprotischen organischen Lösungsmittel. Die Umsetzung vollzieht sich hierbei nach der allgemeinen Reaktionsgleichung:

$$(R^1R^2)NH_2X + MeOCH=CR^3\text{-}COOR^4 \rightarrow (R^1R^2)NCH=CR^3\text{-}COOR^4 + MeX + H_2O.$$

β-Amino-acrylsäureester werden bei der Synthese pharmazeutisch wirksamer 4-Hydroxy-chinolinderivate eingesetzt.

Es wurde ein Verfahren zur Herstellung von β-Amino-acrylsäureestern der Formel

$$(R^1R^2)NCH=CR^3\text{-}COOR^4 \qquad (I)$$

durch Umsetzung von β-Hydroxy-acrylsäureester-alkalisalzen der Formel

$$Me^\oplus \ {}^\ominus OCH=CR^3\text{-}COOR^4 \qquad (II)$$

mit Ammoniumsalzen der allgemeinen Formel

$$(R^1R^2)NH_2{}^\oplus \ X^\ominus \qquad (III)$$

in denen,

$R^1$, $R^2$ und $R^3$     unabhängig voneinander Wasserstoff, geradkettiges oder verzweigtes $C_1$-$C_8$-Alkyl, geradkettiges oder verzweigtes $C_2$-$C_8$-Alkenyl, $C_3$-$C_8$-Cycloalkyl, $C_6$-$C_{12}$-Aryl oder ein 5-7-gliedriges aromatisches oder nicht-aromatisches heterocyclisches System mit 1 oder 2 Heteroatomen aus der Gruppe n und/oder S und/oder O bedeuten,

$R^1$ und $R^2$     gemeinsam mit dem durch sie substituierten n-Atom ein aromatisches oder nicht-aromatisches heterocyclisches System bilden können, das neben dem n-Atom ein weiteres Heteroatom aus der Gruppe N und/oder S und/oder O enthalten kann,

$R^4$     für geradkettiges oder verzweigtes $C_1$-$C_8$-Alkyl steht,

$Me^\oplus$     für ein Alkalimetallion steht und

$X^\ominus$     das Anion einer anorganischen oder organischen Säure bedeutet,

gefunden, das dadurch gekennzeichnet ist, daß die Reaktion bei 0-60°C in einem aprotischen organischen Lösungsmittel als Reaktionsmedium, in dem die Reaktionskomponenten suspendiert vorliegen, durchgeführt wird, wobei ein Teil des aprotischen Lösungsmittels durch ein protisches organisches Lösungsmittel, das mit dem aprotischen Lösungsmittel zu einer homogenen Phase mischbar ist, so ersetzt sein kann, daß das Verhältnis des aprotischen zum protischen Lösungsmittel 1:10-10:1 Gewichtsteile beträgt.

Geradkettiges oder verzweigtes $C_1$-$C_8$-Alkyl ist beispielsweise Methyl, Ethyl, Propyl, Isopropyl, Butyl, Isobutyl, tert.-Butyl, die isomeren Pentyle, Hexyle oder Octyle. In bevorzugter Weise seien die genannten $C_1$-$C_4$-Alkylreste, besonders bevorzugt Methyl oder Ethyl und ganz besonders bevorzugt Methyl genannt.

Geradkettiges oder verzweigtes $C_2$-$C_8$-Alkenyl ist Vinyl, Propenyl, Allyl, Butenyl-1, Butenyl-2, die isomeren Pentenyle, Hexenyle und Octenyle. Bevorzugt sei $C_2$-$C_4$-Alkenyl genannt.

$C_3$-$C_8$-Cycloalkyl ist beispielsweise Cyclopropyl, Cyclobutyl, Cyclopentyl, Methyl-cyclopentyl, Dimethyl-cyclopentyl, Cyclohexyl, Methyl-cyclohexyl, Dimethyl-cyclohexyl, Cycloheptyl und Cyclooctyl. Bevorzugtes Cycloalkyl ist Cyclopropyl, Cyclopentyl und Cyclohexyl.

$C_6$-$C_{12}$-Aryl ist beispielsweise Phenyl, Naphthyl oder Biphenylyl, bevorzugt Phenyl.

Als 5-7-gliedriges, aromatisches oder nicht-aromatisches heterocyclisches System mit 1 oder 2 Heteroatomen aus der Gruppe n und/oder S und/oder O seien beispielsweise genannt: die an der 2-, 3- oder 4-Position verknüpften Reste von Pyrrol, Pyrrolin, Pyrrolidin, Furan, Tetrahydrofuran, Thiophen, Tetrahydrothiophen, Pyrazol, Imidazol, Pyrazolin, Imidazolin, Oxazol, Thiazol, Oxazolin, Thiazolin, Pyridin, Pyran, Thiopyran, Piperidin, Pyridazin, Pyrimidin, Pyrazin, Oxazin, Thiazin, Azepin, Oxazepin, Thiazepin und Thiazocin. In bevorzugter Weise seien 5- oder 6-gliedrige aromatische oder nicht-aromatische heterocyclische Systeme der genannten Art verstanden, in denen wenigstens eines der Heteroatome N ist.

Die genannten Reste können ihrerseits durch $C_1$-$C_4$-Alkyl, bevorzugt Methyl, durch $C_1$-$C_4$-Alkoxy, bevorzugt Methoxy, durch Halogen, wie Chlor, Fluor oder Brom, durch Phenyl oder Hydroxy substituiert sein. Durch die Phenyl-Substitution von Alkyl gelangt man beispielsweise in die Reihe von Aralkyl-Resten. Die aromatischen Teile der genannten Reste und Substituenten können weiterhin durch typisch aromatische Substituenten, wie Nitro, Cyano substituiert sein. Die heterocyclischen Reste können mit einem Benzolkern kondensiert sein.

Für $R^1$ und $R^2$ sei bevorzugt geradkettiges oder verzweigtes $C_1$-$C_4$-Alkyl, besonders bevorzugt $C_1$-$C_2$-Alkyl und ganz besonders bevorzugt Methyl genannt. Für $R^3$ sei bevorzugt Wasserstoff oder geradkettiges oder verzweigtes $C_1$-$C_4$-Alkyl, besonders bevorzugt Wasserstoff genannt. Für $R^4$ sei bevorzugt geradkettiges oder verzweigtes $C_1$-$C_4$-Alkyl, besonders bevorzugt Methyl oder Ethyl genannt.

$Me^\oplus$ ist ein Alkalimetallion, beispielsweise das Kation von Li, Na, K, Rb oder Cs, bevorzugt von Na oder K, besonders bevorzugt von Na.

$X^{\ominus}$ ist das Anion einer anorganischen oder organischen Säure. Als anorganische Säure kommt beispielsweise HF, HCl, HBr, $H_2SO_4$, $HNO_3$, $H_3PO_4$, und als organische Säure eine der niederen Carbonsäuren, wie Ameisensäure, Essigsäure, Propionsäure oder Buttersäure, ferner halogenierte niedere Carbonsäuren, wie Chloressigsäure, Dichloressigsäure, Trichloressigsäure, Trifluoressigsäure und ähnliche, sowie eine Sulfonsäure, wie Methansulfonsäure, Benzolsulfonsäure oder Toluolsulfonsäure, in Betracht. Bevorzugt sei eine der genannten anorganischen Säuren, besonders bevorzugt HCl genannt.

Die erfindungsgemäß umzusetzenden β-Hydroxy-acrylsäureester-alkalisalze mit dem Trivialnamen "($R^3$-substituierte) Formylessigsäureester-alkalisalze" können beispielsweise durch die Einwirkung von CO auf Alkalimetallalkoholat und ($R^3$-substituiertes) Alkylacetat hergestellt werden.

Die Herstellung von N,N-Dimethyl-β-amino-acrylsäure-ethylester aus Natrium-formylessigsäure-ethylester und Dimethylamin-hydrochlorid ist aus Annales de Chimie, 10. Serie, Bd. 18 (1932) 107, besonders 108, bekannt. Hierbei wird in absolutem Ethanol in einer 7-8-stündigen Reaktion eines Ausbeute von 41 % der theoretischen Ausbeute erreicht. Die Isolierung des gewünschten Produkts ist schwierig und verlustreich.

In dem in DE-OS 35 31 067 beschriebenen Verfahren zum gleichen Endprodukt, wie oben beschrieben, wird die Ausbeute dadurch auf ungefähr 75 % der theoretischen Ausbeute gesteigert, daß in wäßriger Lösung des Dialkylamin-hydrochlorids gearbeitet wird; die Reaktionszeit kann hierbei auf ungefähr 2 Stunden gesenkt werden. Aber auch bei diesem Verfahren ist es schwierig, die β-Amino-acrylsäureester ohne größere Verluste aus dem Reaktionsgemisch zu isolieren. Dies gilt insbesondere für Verbindungen mit kleinen Alkylresten, die relativ gut wasserlöslich sind und in größerer Menge im Reaktionsmedium zurückgehalten werden, wie es beispielsweise beim N,N-Dimethyl-β-amino-acrylsäuremethylester geschieht. Das in DE-OS '067 beschriebene Verfahren hat den weiteren Nachteil, daß Wasser ein Reaktionsmedium ist, in welchem die Ausgangsverbindungen schnell zersetzt werden, ganz besonders unter leicht sauren Bedingungen, so daß der Kontakt der Alkali-hydroxy-acrylsäureester mit dem Wasser auf ein Minimum beschränkt werden soll. Die Folge ist, daß der Verfahrensspielraum stark eingeschränkt ist, was in vielen Fällen ein aufwendigeres Vorgehen erforderlich macht. So liegt die Dosierungsweise fest, und es ist gemäß dieser DE-OS beispielsweise nicht möglich, die Reaktion zum β-Amino-acrylsäureester mit der Vorstufe zu einer Eintopfreaktion zusammenzufassen, in dem der Alkali-hydroxy-acrylsäureester ohne Zwischenisolierung durch Zugabe des Dimethylamin-hydrochlorids weiter zum β-Amino-acrylsäureester umgesetzt wird, da hierbei gemäß dem Verfahren von DE-OS '067 Ausbeuteverluste nicht vermieden werden können. Diese zuletztgenannte Dosierungsweise unter Zusammenfassung von zwei Stufen zu einer Eintopfreaktion wird daher auch in dieser DE-OS als negativ bezeichnet.

Erfindungsgemäß können nun die β-Amino-acrylsäureester (I) nach dem obigen allgemeinen Reaktionsschema in hoher Reinheit, in nahezu quantitativen Ausbeuten und bei kurzen Reaktionszeiten dadurch erhalten werden, daß die Reaktion in einem aprotischen organischen Lösungsmittel als Reaktionsmedium, in dem die Reaktionskomponenten suspendiert vorliegen, durchgeführt wird, wobei ein Teil des aprotischen organischen Lösungsmittels durch ein protisches organisches Lösungsmittel, das mit dem aprotischen Lösungsmittel zu einer homogenen Phase mischbar ist, ersetzt sein kann. Hierbei ist ein großer Verfahrensspielraum und eine einfache Isolierung möglich. So betragen die Ausbeuten an β-Amino-acrylsäureester, bezogen auf eingesetztes β-Hydroxy-acrylsäureester-alkalisalz in der Regel über 90 % der theoretischen Ausbeute an mindestens 99 %igem Reaktionsprodukt.

Als aprotische organische Lösungsmittel kommen beispielsweise Benzolabkömmlinge, aliphatische Kohlenwasserstoffe, Ester, Nitrile, Amide und Ether oder Gemische daraus in Frage.

Benzolabkömmlinge sind beispielsweise das Benzol selbst, ferner kommen mit 1 bis 3 $C_1$-$C_4$-Alkylgruppen und/oder 1 bis 3 Alkoxygruppen und/oder 1 bis 2 Halogenatomen, wie Chlor oder Brom substituierte Benzole in Betracht; bei den substituierten Benzolen handelt es sich insbesondere um solche, deren Substituenten 1 bis 6 zusätzliche Atome umfassen. Beispiele hierfür sind: Benzol, Toluol, Xylole, Cumol, Ethylbenzol, Chlorbenzol, Chlortoluol, Dichlorbenzol u.a..

Aliphatische Kohlenwasserstoffe als Reaktionsmedien können geradkettig oder verzweigt sein und 5-10 C-Atome enthalten und in reiner Form oder als Gemisch vorliegen. Beispiele sind etwa Petrolether und Ligroin.

Ester sind beispielsweise Essigsäureethylester, Propionsäuremethylester u.ä.; Nitrile sind beispielsweise Acetonitril, Benzonitril u.ä.; Amide sind beispielsweise Dimethylformamid, Dimethylacetamid u.ä.

Als geeignete Ether seien offenkettige oder cyclische Verbindungen mit 4-8 C-Atomen genannt, wie Tetrahydrofuran, Dioxan, Dibutylether, Methyl-tert.- butylether und ähnliche.

Für eine einfache destillative Isolierung sollte der Siedepunkt des gewählten Lösungsmittels den Siedepunkt der gewünschten Verbindung entweder deutlich unter- oder überschreiten.

In bevorzugter Weise werden Toluol, Xylol, Essigsäureester oder ein Gemisch von zwei oder allen dieser Stoffe als aprotisches Lösungsmittel eingesetzt.

Als protisches organisches Lösungsmittel kommen in Frage: $C_1$-$C_8$-Alkanole, Phenol, durch Methylgrup-

pen, Ethylgruppen, Chlor und/oder Brom substituiertes Phenol oder Gemische daraus. Solche protischen Lösungsmittel haben einen pKs-Wert, der größer als 10 ist. In bevorzugter Weise wird ein Alkanol, besonders bevorzugt ein $C_1$-$C_4$-Alkanol, ganz besonders bevorzugt Methanol und/oder Ethanol eingesetzt.

Das Verhältnis des aprotischen zum protischen Lösungsmittel beträgt 1:10 - 10:1 Gewichtsteile, bevorzugt 1:2 - 7:1 Gewichtsteile.

Die Menge des eingesetzten aprotischen organischen Lösungsmittels oder eines Gemisches aus aprotischem und protischem Lösungsmittel ist lediglich so auszuwählen, daß mit dem in der Reaktionsapparatur vorhandenen Rührer jederzeit eine gute Durchmischung der Suspension gewährleistet ist und das entstehende Reaktionsprodukt am Ende der Reaktion möglichst vollständig in gelöster Form in diesem Lösungsmittel vorliegt. Hierzu sei beispielsweise eine Menge von 130 Gew.-% bis 500 Gew.-% Lösungsmittel-(gemisch) bezogen auf eingesetztes β-Hydroxy-acrylsäureester-alkalisalz, genannt.

Die Reaktionskomponenten (I) und (II) können in stöchiometrischen Mengen eingesetzt werden. Mit Vorteil wird jedoch ein Überschuß der billigeren Komponente (im allgemeinen das Ammoniumsalz) von 1-80 Mol-%, bezogen auf die teurere Komponente, eingesetzt. In bevorzugter Weise wird daher meist ein Ammoniumsalzüberschuß, beispielsweise von 7-30 Mol-%, eingesetzt. Das molare Verhältnis von β-Hydroxy-acrylsäureester-alkalisalz zu Ammoniumsalz beträgt daher 1:0,7-1,8, bevorzugt 1:1,01-1,8, besonders bevorzugt 1:1,07-1,3.

Die Reaktion verläuft im erfindungsgemäßen Verfahren auch bei niedrigen Temperaturen von 0° C und tiefer ausreichend schnell. Bei Temperaturen über 60°C nimmt die Bildung von Nebenprodukten deutlich zu. Günstige Reaktionstemperaturen liegen daher bei 0-60°C, bevorzugt 15 bis 30°C.

Die Zahl und Bandbreite der möglichen Verfahrensweisen ist wegen der im aprotischen bzw. aprotisch/protischen Reaktionsmedium wesentlich geringeren Zersetzlichkeit der β-Hydroxy-acrylsäureester-alkalisalze groß.

Beispielsweise kann die Reaktion so durchgeführt werden, daß zur Suspension oder Lösung des Ammoniumsalzes in einem erfindungsgemäßen Lösungsmittel das β-Hydroxy-acrylsäureester-alkalisalz in fester Form oder als Suspension in einem erfindungsgemäßen Lösungsmittel unter gutem Rühren hinzugefügt wird. Wird in einem Gemisch aus aprotischem und protischem Lösungsmittel gearbeitet, wird das Ammoniumsalz vorteilhaft als Suspension in einem protischen Lösungsmittel vorgelegt.

Eine andere Verfahrensweise besteht darin, der Suspension des β-Hydroxy-acrylsäureester-alkalisalzes in einem erfindungsgemäßen Lösungsmittel das Ammoniumsalz in fester Form oder als Suspension in einem erfindungsgemäßen Lösungsmittel unter gutem Rühren zuzufügen.

Eine noch weitere Verfahrensvariante besteht darin, der Suspension des β-Hydroxy-acrylsäureester-alkalisalzes in einem erfindungsgemäßen Lösungsmittel das zugehörige Amin, konzentriert oder mit einem der genannten aprotischen oder protischen organischen Lösungsmittel verdünnt, zuzuführen und dann unter gutem Rühren eine dem β-Hydroxy-acrylsäureester-alkalisalz entsprechende Menge einer anorganischen oder organischen Säure, beispielsweise Chlorwasserstoff, zuzufügen. Der Zusatz von Amin und Säure kann auch simultan unter Kontrolle ihres äquivalenten Verhältnisses (z.B. mit einer pH-Elektrode) erfolgen.

In einer bevorzugten Ausführungsform wird das β-Hydroxy-acrylsäureester-alkalisalz bei seiner Herstellung durch Reaktion des entsprechenden Carbonsäureesters mit CO und Alkalimetall-alkoholat in einem erfindungsgemäßen Reaktionsmedium aus aprotischem organischem Lösungsmittel erhalten und liegt im allgemeinen als Suspension vor. Zu dieser Suspension des Alkalisalzes wird nun das Ammoniumsalz in fester Form und gegebenenfalls zusätzlich protisches Lösungsmittel oder besonders bevorzugt als Suspension in einem aprotischen und/oder protischen Lösungsmittel oder, wie oben bereits beschrieben, zuerst als das reine Amin (gegebenenfalls verdünnt mit einem der genannten Lösungsmittel) und anschließendem Zusatz der Säure unter gutem Rühren versetzt.

In einer anderen bevorzugten Ausführungsform wird die aus der Vorstufe in einem aprotischen Lösungsmittel erhaltene Suspension zu einer Suspension des Ammoniumsalzes in einem aprotischen und/oder protischen Lösungsmittel hinzugefügt. Die β-Amino-acrylsäureester sind nach einer solchen Verfahrensweise in einer Eintopfreaktion mit Gesamtausbeuten von 80-95 %, bezogen auf den eingesetzten Carbonsäureester bzw. bezogen auf eingesetztes Alkalimetall-alkoholat, herstellbar.

Im Gegensatz zu dem bekannten Verfahren ist die Abtrennung des Reaktionsproduktes einfach und gelingt, der Ausbeute nach zu urteilen, praktisch quantitativ. Nachdem das Reaktionswasser durch Andestillieren azeotrop entfernt ist, wobei eventuell mitverwendetes protisches Lösungsmittel teilweise mit übergeht, kann das gebildete Alkalisalz der anorganischen oder organischen Säure gut abfiltriert werden. Die bei den bekannten Verfahren beobachtete Zurückhaltung von Reaktionsprodukt in abzutrennenden Lösungsmitteln tritt im erfindungsgemäßen Verfahren nicht auf. Die erfindungsgemäß einzusetzenden organischen Lösungsmittel erweisen sich bei der sich an die Salzabtrennung anschließenden destillativen Aufarbeitung als inert bezüglich des Reaktionsproduktes. So verbleibt typischerweise nach der destillativen Entfernung des Lösungsmittels und der ebenfalls destillativen Gewinnung des Reaktionsproduktes kaum ein Rückstand, der auf wesentliche Mengen an Nebenprodukten schließen ließe. Ohne weitere Feindestillation gewinnt man so ein sehr reines Produkt mit mehr als 98 %, vielfach mehr als 99 % Reinheit.

Beispiel 1

β-(N,N-Dimethylamino)-acrylsäuremethylester

In 120 ml Toluol wurden bei 0 bis 5°C 27,5 g (0,61 Mol) kondensiertes Dimethylamin eingetropft. Anschließend wurden unter Kühlung bei der gleichen Temperatur 21,9 g (0,60 Mol) Chlorwasserstoff eingeleitet. Unter Erwärmung auf 20 bis 25°C wurden 74,4 g 90,0 %iger Natrium-formyl-essigsäure-methylester (0,54 Mol) unter gutem Rühren als Feststoff im Verlauf einer halben Stunde eingetragen. Durch leichtes Kühlen mit einem Eisbad wurde die schwach exotherme Reaktion auf einer Temperatur von ungefähr 25°C gehalten und das Gemisch eine Stunde nachgerührt. Die anfangs dicke Suspension wurde während der Zugabe der ersten Hälfte des Natrium-formylessigsäureesters zunehmend dünnflüssiger und ihre Farbe wechselte von Beige nach Gelborange. Am Rotationsverdampfer wurden aus der Suspension niedrigsiedende Komponenten und etwa 80 bis 90 % des Toluols bei leichtem Vakuum (ca. 300 mbar) abdestilliert. Dabei wurde auch das Reaktionswasser aus dem Gemisch azeotrop entfernt. Das gebildete Kochsalz und der Überschuß an Dimethylammoniumchlorid wurden abgesaugt und mit 50 ml Toluol gewaschen. Die vereinigten Filtrate wurden im Vakuum ohne Kolonne destilliert. Nach einem Vorlauf aus restlichem Toluol gingen bei 1,0 mbar und einer Kopftemperatur von 65 bis 70°C 69,0 g N,N-Dimethyl-β-aminoacrylsäure-methylester über.

Im Destillationskolben blieb ein Rückstand von 1,5 g, der zum größten Teil aus weiterem Produkt bestand.

Das Produkt ist leicht gelblich gefärbt und kristallisiert nach kurzem Stehen in der Destillationsvorlage. Schmelzpunkt: 48-50°C.

Der gaschromatographisch bestimmte Gehalt betrug 99,6 Gew.-%.

Dies entsprach einer Ausbeute von 98,6 % der theoretischen Ausbeute, bezogen auf eingesetzten Natrium-formylessigsäure-methylester.

Beispiel 2

β-(N,N-Dimethylamino)-acrylsäureethylester

Zu 274,5 g einer toluolischen Suspension, die aus der Herstellung von Natrium-formylessigsäure-ethylester stammte und die 71,2 g (0,515 Mol) Natriumformylessigsäureethylester enthielt, wurden bei 20 bis 25°C 120 ml einer toluolischen Suspension von 50,40 g (0,62 Mol) Dimethylammoniumchlorid unter gutem Rühren im Verlauf einer halben Stunde eingetragen und eine Stunde nachgerührt. Die rot-orange-farbige, dünnflüssige Suspension wurde am Rotationsverdampfer bei leichtem Vakuum (ca. 300 mbar) von niedrigsiedenden Komponenten und von etwa 80 bis 90 % des Toluols einschließlich des azeotrop übergehenden Wassers befreit. Die Feststoffe wurden abgesaugt, mit 50 ml Toluol gewaschen und die vereinigten Filtrate im Vakuum ohne Kolonne destilliert. Nach einem Vorlauf aus Toluol gingen bei 0,6 mbar und einer Kopftemperatur von 94 bis 95°C 85,8 g farbloser N,N-Dimethyl-β-aminoacrylsäure-ethylester über.

Im Destillationskolben blieb ein Rückstand von 2,7 g, der zum größten Teil aus weiterem Produkt bestand.

Der gaschromatographisch bestimmte Gehalt betrug 99,8 Gew.-%.

Dies entsprach einer Ausbeute von 96,8 % der theoretischen Ausbeute, bezogen auf eingesetzten Natrium-formylessigsäure-ethylester.

Beispiel 3

β-(N,N-Dimethylamino)-acrylsäureethylester

Eine Suspension von 34,0 g (0,5 mol) Natriumethylat in 200 ml Toluol und 66,1 g (0,75 mol) Essigsäureethylester wurde in einem 0,7 Ltr.-Autoklaven vorgelegt. Nach Spülung mit Stickstoff und anschließend mit Kohlenmonoxid wurde über das Reduzierventil ein CO-Druck von 20 bar eingestellt und auf 60°C aufgeheizt.

Temperatur und Druck wurden konstant gehalten. Nach 5 Stunden war die CO-Aufnahme beendet.

Nach dem Abkühlen auf Raumtemperatur wurde die dicke, beigefarbene Suspension bei Raumtemperatur unter gutem Rühren im Verlauf einer halben Stunde in eine vorgelegte Suspension von 40,8 g (0,5 mol) Dimethylammoniumchlorid in 100 ml Toluol eingetropft.

Durch leichtes Kühlen mit einem Eisbad wurde die Temperatur auf ungefähr 25°C gehalten und eine Stunde nachgerührt.

Nachdem das gebildete Kochsalz und der Überschuß an Dimethylammoniumchlorid abgesaugt und mit 50 ml Toluol gewaschen worden waren, wurden die vereinigten Filtrate im Vakuum ohne Kolonne destilliert.

Nach einem Vorlauf aus Toluol gingen bei 0,6 mbar und einer Kopftemperatur von 88 bis 96°C 65,7 g farbloser β-(N,N-Dimethylamino)-acrylsäureethylester über. Im Destillationskolben blieb ein Rückstand von 2,1 g.

Der gaschromatographisch bestimmte Gehalt betrug 98,9 Gew.-%. Dies entspricht einer Ausbeute von 90,8 % d. Th., bezogen auf eingesetztes Natriumethylat.

Beispiel 4

Die Suspension von 68 g (1 mol) 93 proz. Natriummethylat in 132 g (1,5 mol) Essigsäureethylester und 300 ml Toluol wurde in einem 1 Ltr.-Autoklaven vorgelegt. Nach Aufheizen auf 50°C wurde ein CO-Druck von 20 bar eingestellt. 4 h später war die CO-Aufnahme beendet und man ließ abkühlen.

Der Autoklaveninhalt wurde nun bei Raumtemperatur zu einer Suspension von 81,6 g (1 mol) Dimethylaminhydrochlorid in 70 ml Methanol getropft. Nach 2 h Nachrühren saugte man ab, wusch mit 30 ml Toluol nach und engte das Filtrat am Rotationsverdampfer ein. Bei der anschließenden Vakuumdestillation bei 0,7 mbar und 83-89°C wurden 123,5 g 98,6 %iges β-(N,N-Dimethylamino)-acrylsäure-ethylester erhalten, was einer Ausbeute von 94,2 % über beide Stufen entspricht. Der Rückstand bestand aus 3,3 g braun gefärbtem Salz.

Beispiel 5

Eine Suspension aus 54 g (1 mol) 91,5 proz. Natriummethylat in 111 g (1,5 mol) Essigsäuremethylester und 250 ml Toluol wurde auf 80°C aufgeheizt und ein CO-Druck von 40 bar eingestellt. Nachdem die CO-Aufnahme beendet war (4 h), ließ man abkühlen und tropfte die entstandene Suspension zu 81,6 g (1 mol) Dimethylaminhydrochlorid in 100 ml Methanol. Nach 2 h Nachrühren wurde der Ansatz auf die Hälfte eingeengt. Man filtrierte vom Salz, wusch mit 30 ml Toluol und destillierte das Lösungsmittel vom Filtrat ab. Nachfolgende Vakuumdestillation lieferte 105,5 g 99,6 proz. Produkt, was einer Ausbeute von 89 % über beide Stufen entspricht.

Beispiel 6

In einem Gemisch aus 50 ml Toluol und 50 ml Ethanol wurden 24 g (0,29 mol) Dimethylaminhydrochlorid vorgelegt. Mit Hilfe eines Feststoffdosierers fügte man innerhalb 10 min. 40 g (0,29 mol) 92,6 proz. Natriumformylessigsäureethylester zu. Nach 2 h Nachrühren bei 20°C wurde abgesaugt. Die Aufarbeitung lieferte 35,0 g 98,4 proz. Produkt, was einer Ausbeute von 92,7 % entspricht.

Beispiel 7

Der Ansatz aus Beispiel 6 wurde in einem Lösungsmittelgemisch aus 54 ml Toluol, 36 ml Ethanol und 16 ml Essigsäureethylester durchgeführt. Man erhielt eine Ausbeute von 88,7 %.

Beispiel 8

In eine Suspension von 40 g (0,29 mol) Natriumformyl-essigsäureethylester in 50 ml Toluol und 50 ml Ethanol wurden 13,1 g (0,29 mol) Dimethylamin eingeleitet. Anschließend leitete man bis zum Erreichen des Neutralpunktes HCl-Gas eine und rührt 2 h bei 20°C nach. Nach dem Absaugen wurde wie üblich aufgearbeitet. Man erhielt β-Dimethylaminoacrylsäureethylester in einer Ausbeute von 91,6 %.

**Patentansprüche**

1.  Verfahren zur Herstellung von β-Amino-acrylsäureestern der Formel
$$(R^1R^2)NCH=CR^3\text{-}COOR^4$$
durch Umsetzung von β-Hydroxy-acrylsäureesteralkalisalzen der Formel
$$Me^{\oplus}\;^{\ominus}OCH=CR^3\text{-}COOR^4$$
mit Ammoniumsalzen der allgemeinen Formel
$$(R^1R^2)NH_2^{\oplus}\;X^{\ominus},$$
in denen

R$^1$, R$^2$ und R$^3$        unabhängig voneinander Wasserstoff, geradkettiges oder verzweigtes $C_1$-$C_8$-Alkyl,

geradkettiges oder verzweigtes $C_2$-$C_8$-Alkenyl, $C_3$-$C_8$-Cycloalkyl, $C_6$-$C_{12}$-Aryl oder ein 5-7-gliedriges aromatisches oder nicht-aromatisches heterocyclisches System mit 1 oder 2 Heteroatomen aus der Gruppe N und/oder S und/oder O bedeuten,

$R^1$ und $R^2$ gemeinsam mit dem durch sie substituierten N-Atom ein aromatisches oder nichtaromatisches heterocyclisches System bilden können, das neben dem N-Atom ein weiteres Heteroatom aus der Gruppe N und/oder S und/oder O enthalten kann,

$R^4$ für geradkettiges oder verzweigtes $C_1$-$C_8$-Alkyl steht,

$Me^{\oplus}$ für ein Alkalimetallion steht und

$X^{\ominus}$ das Anion einer anorganischen oder organischen Säure bedeutet,

dadurch gekennzeichnet, daß die Reaktion bei 0-60°C in einem aprotischen organischen Lösungsmittel als Reaktionsmedium, in dem die Reaktionskomponenten suspendiert vorliegen, durchgeführt wird, wobei ein Teil des aprotischen organischen Lösungsmittels durch ein protisches organisches Lösungsmittel, das mit dem aprotischen Lösungsmittel zu einer homogenen Phase mischbar ist, so ersetzt sein kann, daß das Verhältnis des aprotischen zum protischen Lösungsmittel 1:10-10:1 Gewichtsteile beträgt.

2. Verfahren nach Anspruch 1, dadurch gekennzeichnet, daß $R^1$ und $R^2$ für geradkettiges oder verzweigtes $C_1$-$C_4$-Alkyl, bevorzugt für $C_1$-$C_2$-Alkyl und besonders bevorzugt für Methyl steht.

3. Verfahren nach Anspruch 1, dadurch gekennzeichnet, daß $R^3$ für Wasserstoff oder geradkettiges oder verzweigtes $C_1$-$C_4$- Alkyl, bevorzugt für Wasserstoff steht.

4. Verfahren nach Anspruch 1, dadurch gekennzeichnet, daß $R^4$ geradkettiges oder verzweigtes $C_1$-$C_4$-Alkyl, bevorzugt Methyl oder Ethyl bedeutet.

5. Verfahren nach Anspruch 1, dadurch gekennzeichnet, daß $Me^{\oplus}$ für $Na^{\oplus}$ oder $K^{\oplus}$, bevorzugt für $Na^{\oplus}$ steht und daß $X^{\ominus}$ das Anion einer anorganischen Säure, bevorzugt das Chloridanion ist.

6. Verfahren nach Anspruch 1, dadurch gekennzeichnet, daß als aprotisches Lösungsmittel Toluol, Xylol, Essigsäureester oder ein Gemisch von zwei oder allen dieser Stoffe und als protisches Lösungsmittel ein geradkettiger oder verzweigter $C_1$-$C_4$-Alkohol, bevorzugt Methanol oder Ethanol, eingesetzt werden.

7. Verfahren nach Anspruch 1, dadurch gekennzeichnet, daß das molare Verhältnis von β-Hydroxy-acrylsäureester-alkalisalz zu Ammoniumsalz 1:0,7-1,8, bevorzugt 1:1,01-1,8, besonders bevorzugt 1:1,07-1,3 beträgt.

8. Verfahren nach Anspruch 1, dadurch gekennzeichnet, daß als β-Hydroxy-acrylsäureester-alkalisalz die aus seiner Herstellung aus Carbonsäureester, Alkalimetall-alkoholat und CO in einem aprotischen organischen Lösungsmittel vorliegende Suspension eingesetzt wird, zu der das Ammoniumsalz in fester form, bevorzugt als Suspension in einem aprotischen oder protischen organischen Lösungsmittel oder einem Gemisch solcher Lösungsmittel, oder getrennt zunächst als reines oder mit einem aprotischen oder protischen organischen Lösungsmittel verdünntes Amin, gefolgt von einer anorganischen oder organischen Säure, bevorzugt HCl, zugegeben wird.

9. Verfahren nach Anspruch 1, dadurch gekennzeichnet, daß die aus Carbonsäureester, Alkalimetall-alkoholat und CO in einem aprotischen organischen Lösungsmittel erhaltene Suspension zu einer Suspension des Ammoniumsalzes in einem aprotischen oder protischen organischen Lösungsmittel hinzugefügt wird.

## Claims

1. Process for the preparation of β-amino-acrylic acid esters of the formula

$$(R^1R^2)NCH=CR^3\text{-}COOR^4$$

by reaction of β-hydroxy-acrylic acid ester alkali metal salts of the formula

$$Me^{\oplus} {}^{\ominus}OCH=CR^3\text{-}COOR^4$$

with ammonium salts of the general formula

$$(R^1R^2)NH_2^{\oplus} X^{\ominus},$$

in which

$R^1$, $R^2$ and $R^3$ independently of one another denote hydrogen, straight-chain or branched $C_1$-$C_8$-alkyl, straight-chain or branched $C_2$-$C_8$-alkenyl, $C_3$-$C_8$-cycloalkyl, $C_6$-$C_{12}$-aryl or a 5-7-membered aromatic or non-aromatic heterocyclic system having 1 or 2 heteroatoms from the group comprising N and/or S and/or O,

$R^1$ and $R^2$, together with the N atom substituted by them, can form an aromatic or non-aromatic heterocyclic system which, in addition to the N atom, can contain a further heteroatom from the group comprising N and/or S and/or O,

$R^4$ represents straight-chain or branched $C_1$-$C_8$-alkyl,

$Me^\oplus$ represents an alkali metal ion and

$X^\ominus$ denotes the anion of an inorganic or organic acid, characterized in that the reaction is carried out at 0-60°C in an aprotic organic solvent, in which the reaction components are suspended, as the reaction medium, it being possible to replace a part of the aprotic solvent by a protic organic solvent which is miscible with the aprotic solvent to give a homogeneous phase such that the ratio of the aprotic solvent to the protic solvent is 1:10-10:1 parts by weight.

2. Process according to Claim 1, characterized in that $R^1$ and $R^2$ represent straight-chain or branched $C_1$-$C_4$-alkyl, preferably $C_1$-$C_2$-alkyl and particularly preferably methyl.

3. Process according to Claim 1, characterized in that $R^3$ represents hydrogen or straight-chain or branched $C_1$-$C_4$-alkyl, preferably hydrogen.

4. Process according to Claim 1, characterized in that $R^4$ denotes straight-chain or branched $C_1$-$C_4$-alkyl, preferably methyl or ethyl.

5. Process according to Claim 1, characterized in that $Me^\oplus$ represents $Na^\oplus$ or $K^\oplus$, preferably $Na^\oplus$ and in that $X^\ominus$ is the anion of an inorganic acid, preferably the chloride anion.

6. Process according to Claim 1, characterized in that toluene, xylene, ethyl acetate or a mixture of two or all of these substances is employed as the aprotic solvent and a straight-chain or branched $C_1$-$C_4$-alcohol, preferably methanol or ethanol, is employed as the protic solvent.

7. Process according to Claim 1, characterized in that the molar ratio of β-hydroxy-acrylic acid ester alkali metal salt to ammonium salt is 1:0.7-1.8, preferably 1:1.01-1.8, particularly preferably 1:1.07-1.3.

8. Process according to Claim 1, characterized in that, as the β-hydroxy-acrylic acid ester alkali metal salt, the suspension present from its preparation from carboxylic acid ester, alkali metal alkoxide and CO in an aprotic organic solvent is employed, to which the ammonium salt is added in solid form, preferably as a suspension in an aprotic or protic organic solvent or a mixture of such solvents, or separately initially as pure amine or amine diluted with an aprotic or protic organic solvent, followed by an inorganic or organic acid, preferably HCl.

9. Process according to Claim 1, characterized in that the suspension obtained from carboxylic acid ester, alkali metal alkoxide and CO in an aprotic organic solvent is added to a suspension of the ammonium salt in an aprotic or protic organic solvent.

## Revendications

1. Procédé de préparation des β-amino-acrylates de formule :
$$(R^1R^2)NCH=CR^3\text{-}COOR^4$$
par réaction de sels alcalins de β-hydroxy-acrylate de formule :
$$Me^\oplus {}^\ominus OCH=CR^3\text{-}COOR^4$$
avec des sels d'ammonium de formule générale :
$$(R^1 R^2)NH_2^\oplus X^\ominus,$$
dans lesquelles

$R^1$, $R^2$ et $R^3$ représentent indépendamment les uns des autres un atome d'hydrogène, un groupement alkyle en $C_1$-$C_8$ linéaire ou ramifié, un groupement alcényle en $C_2$-$C_8$ linéaire ou ramifié, un groupement cycloalkyle en $C_3$-$C_8$, un groupement aryle en $C_6$-$C_{12}$ ou un système hétérocyclique aromatique ou non aromatique ayant de 5 à 7 chaînons et 1 ou 2 hétéroatomes du groupe constitué par N et/ou S et/ou O,

$R^1$ et $R^2$ ensemble avec l'atome N auquel ils sont substitués peuvent former un système hétérocyclique aromatique ou non aromatique, qui en plus de l'atome N, peut contenir un autre hétéroatome du groupe constitué de N et/ou S et/ou O,

$R^4$ représente un groupement alkyle en $C_1$-$C_8$ linéaire ou ramifié,

$Me^{\oplus}$ représente l'ion d'un métal alcalin et

$X^{\ominus}$ représente l'anion d'un acide inorganique ou organique,

caractérisé en ce que la réaction est effectuée à une température comprise entre 0 et 60°C dans un solvant organique aprotique comme milieu réactionnel dans lequel les composés de la réaction sont présents en suspension, une partie du solvant aprotique pouvant être remplacée par un solvant organique protique miscible avec le solvant aprotique en une phase homogène, de telle façon que le rapport du solvant aprotique au solvant protique est de 1 : 10 à 10 : 1 parties en poids.

2. Procédé selon la revendication 1, caractérisé en ce que $R^1$ et $R^2$ représentent des groupements alkyle en $C_1$-$C_4$ linéaires ou ramifiés, de préférence des groupements alkyle en $C_1$-$C_2$ et encore mieux le groupement méthyle.

3. Procédé selon la revendication 1, caractérisé en ce que $R^3$ représente un atome d'hydrogène ou un groupement alkyle en $C_1$-$C_4$ linéaire ou ramifié, de préférence un atome d'hydrogène.

4. Procédé selon la revendication 1, caractérisé en ce que $R^4$ représente un groupement alkyle en $C_1$-$C_4$ linéaire ou ramifié, de préférence le groupement méthyle ou le groupement éthyle.

5. Procédé selon la revendication 1, caractérisé en ce que $Me^{\oplus}$ représente $Na^{\oplus}$ ou $K^{\oplus}$, de préférence $Na^{\oplus}$ et que $X^{\ominus}$ est l'anion d'un acide inorganique, de préférence l'ion chlorure.

6. Procédé selon la revendication 1, caractérisé en ce qu'on utilise comme solvant aprotique le toluène, le xylène, un ester de l'acide acétique ou un mélange de deux de ces produits ou de tous ces produits et comme solvant protique un alcool en $C_1$-$C_4$ linéaire ou ramifié, de préférence le méthanol ou l'éthanol.

7. Procédé selon la revendication 1, caractérisé en ce que le rapport molaire du sel alcalin de β-hydroxy-acrylate au sel d'ammonium est de 1 : 0,7 à 1,8, de préférence de 1 : 1,01-1,8, encore mieux de 1 : 1,07-1,3.

8. Procédé selon la revendication 1, caractérisé en ce qu'on utilise comme sel alcalin de β-hydroxy-acrylate la suspension obtenue lors de sa préparation à partir d'un ester d'acide carboxylique, d'un alcoolate de métal alcalin et de CO dans un solvant organique aprotique, qu'on lui ajoute le sel d'ammonium sous forme solide, de préférence sous forme de suspension dans un solvant organique aprotique ou protique ou un mélange de tels solvants ou séparément tout d'abord sous forme d'amine pure ou en diluée dans un solvant organique aprotique ou protique et qu'on ajoute ensuite un acide organique ou inorganique, de préférence HCl.

9. Procédé selon la revendication 1, caractérisé en ce qu'on ajoute la suspension obtenue à partir d'un ester d'acide carboxylique, d'un alcoolate de métal alcalin et de CO dans un solvant organique aprotique à une suspension du sel d'ammonium dans un solvant organique aprotique ou protique.